**Europäisches Patentamt**

**European Patent Office**

**Office européen des brevets**

(19)

(11) Veröffentlichungsnummer: **0 179 332 B1**

# EUROPÄISCHE PATENTSCHRIFT

(12)

(45) Veröffentlichungstag der Patentschrift: **13.01.93**

(51) Int. Cl.⁵: **C07K 5/00**, C07K 7/00, A61K 37/02

(21) Anmeldenummer: **85112579.9**

(22) Anmeldetag: **04.10.85**

(54) Neue ZNS-aktive Peptide mit Wirkung auf das cholinerge System.

(30) Priorität: **22.10.84 US 663193**

(43) Veröffentlichungstag der Anmeldung:
**30.04.86 Patentblatt 86/18**

(45) Bekanntmachung des Hinweises auf die
Patenterteilung:
**13.01.93 Patentblatt 93/02**

(84) Benannte Vertragsstaaten:
**AT BE CH DE FR GB IT LI LU NL SE**

(56) Entgegenhaltungen:
US-A- 3 842 064
US-A- 3 853 838
US-A- 4 104 371

Ann. N.Y. A-cad. Sci 297 (1977), 263-74

Life Sci 48 (1991), 155-161

(73) Patentinhaber: **HOECHST AKTIENGESELL-
SCHAFT
Postfach 80 03 20
W-6230 Frankturt am Main 80(DE)**

(72) Erfinder: **Geiger, Rolf, Prof. Dr.
Heinrich-Bleicher-Strasse 33
W-6000 Frankturt am Main 50(DE)**
Erfinder: **Gerhards, Hermann, Dr.
Wacholderweg 4
W-6238 Hofheim am Taunus(DE)**
Erfinder: **Kruse, Hansjörg, Dr.**

**verstorben(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

EP 0 179 332 B1

**Beschreibung**

Wir haben beobachtet, daß schon einfache Dipeptid-Derivate wie z.B.

Z-Lys-Phe-OMe     (IV)

(Z = Benzyloxycarbonyl) nach intracerebroventrikulärer (i.c.v.) Gabe von 10 $\mu$g bei Ratten Putz- und Bewegungsdrang hervorrufen. Gleichzeitig werden cholinerge Mechanismen im Zentralnervensystem (ZNS) beeinflußt. Im Striatum der Ratte ist nach subcutaner Gabe von 10 $\mu$g der Gehalt an Cholin erniedrigt, der von Acetylcholin erhöht.

Die Wirkungen werden verstärkt, wenn die Carboxylgruppe einen basisch substituierten Rest trägt, wobei Lysin in der D-Form verliegen kann. So zeigt z.B. Verbindung V

Z-D-Lys-Phe-NH-$(CH_2)_8$-$NH_2$·2 HCl     (V)

schon mit 1 $\mu$g i.c.v. bzw. s.c. dieselben Wirkungen.

Die beschriebenen Effekte sind für ACTH und MSH charakteristisch.

Wurde in V der Z-Rest durch Phenylalanin ersetzt und dessen $\alpha$-Aminogruppe durch Partialsequenzen von ACTH/MSH aber auch durch andere Acyl-, Aminosäure- und Peptidreste substituiert, konnte eine weitere Steigerung der Effekte im cholinergen System unter Abschwächung des Putz- und Bewegungsdrangs beobachtet werden.

Bei den stärker wirksamen Verbindungen beobachtet man nicht nur erhöhte, sondern auch erniedrigte Acetylcholinspiegel. Beide Effekte weisen auf eine erhöhte Syntheserate bzw. einen erhöhten Umsatz hin.

Eine Beschleunigung des Acetylcholin-Umsatzes in verschiedenen Hirngebieten nach intracerebroventrikulärer (i.c.v)-Gabe von alpha-MSH, ACTH und längerkettigen ACTH-Bruchstücken (ACTH 1-24) wurde bereits früher von zwei Arbeitsgruppen beschrieben. (P. Wood et al.: Life Sciences, 22 (1978), 673-678; JPET, 209 (1979), 97-103; L.J. Botticelli and R.J. Wurtmann: Brain Research 210 (1981), 479-484; J. Neuroscience 2 (1982), 1316-1321).

Neurotrope Wirkung besitzen auch ACTH-ähnliche kurze Peptide die aus Ann. N.Y. Acad. Sci. 297 - (1977) 267-274 bekannt sind.

Bei den obengenannten Versuchen zur Wirkungsverstärkung stellte sich auch heraus, daß die Natur der N-terminalen Substituenten weniger wichtig ist als die Substitution an sich, besonders, wenn es sich bei den Substituenten um Peptide handelt. Auch lassen sich die Phenylalanine ohne Wirkungsverlust modifizieren. Ferner zeigte sich, daß der C-terminale basische Substituent in Vergleich zu einer nichtsubstituierten Verbindung die Wirkung stets um das etwa 100-fache steigert.

Die Erfindung betrifft daher Verbindungen der allgemeinen Formel I

$R^4$-$A^5$-$A^6$-$R^7$     (I)

in welcher bedeuten:

$R^4$ =          über $N_\alpha$ gebundenes Benzyloxycarbonyl (Z),  oder

$R^1$-$A^2$-$A^3$-$A^4$,     worin $A^3$ His oder Ala bedeutet, $A^4$ für Phe steht,

$A^2$          für Glu, oder Ala steht und

$R^1$          für Glutaroyl, H-Met(O), H-Met($O_2$), H-Tyr, oder Pyroglutamyl steht,

$A^5$ =          D-Lys,

$A^6$ =          den Rest von Phenylalanin, und

$R^7$ =          NH-$(CH_2)_n$-$NH_2$,wobei n für eine ganze Zahl von 4-10, steht, wobei H-Met(O)-Glu-His-Phe-D-Lys-Phe-NH-$(CH_2)_8$-$NH_2$ ausgenommen ist, sowie deren physiologisch verträglichen Salze.

Die Erfindung betrifft sowohl Verbindungen mit R- als auch mit S-konfigurierter Sulfinylgruppe des Restes H-Met(O)-.

Die Art des basischen C-terminalen Restes $R^7$ ist unkritisch. Als vorteilhaft haben sich Reste wie -NH-$(CH_2)_n$-$NH_2$ erwiesen, wobei n vorzugsweise 6-10, insbesondere 8 ist.

Nachstehend seien einige Teilsequenzen bevorzugter Penta- und Hexapeptid-Derivate angegeben:

2

```
-Glu-His-Phe-D-Lys-Phe-
-Ala-Ala-Phe-D-Lys-Phe-
-Glu-Ala-Phe-D-Lys-Phe-
```

Die folgenden Verbindungen der Formel I sind besonders bevorzugt:

$$\text{H-Met(O)-Glu-His-Phe-D-Lys-Phe-NH-(CH}_2)_6\text{-NH}_2,$$
$$\text{mit S- bzw. R-konfigurierter Sulfinylgruppe,}$$
$$\text{H-Met (O}_2)\text{-Glu-His-Phe-D-Lys-Phe-NH-(CH}_2)_8\text{-NH}_2,$$
$$\text{H-Met (O}_2)\text{-Ala-Ala-Phe-D-Lys-Phe-NH-(CH}_2)_8\text{-NH}_2 \text{ und}$$
$$\text{HOOC-(CH}_2)_3\text{-CO-Glu-Ala-Phe-D-Lys-Phe-NH-(CH}_2)_8)\text{-NH}_2.$$

Die Erfindung betrifft ferner ein Verfahren zur Herstellung von Verbindungen der Formel I, das dadurch gekennzeichnet ist, daß man ein Fragment mit N-terminaler freier Aminogruppe kondensiert mit einen Fragment mit C-terminaler freier Carboxylgruppe, wobei in diesen Fragmenten an der Reaktion nicht beteiligte primäre und sekundäre Aminogruppen mit sauer abspaltbaren Schutzgruppen vom Urethantyp (wie z.B. Boc) und Carboxylgruppen mit sauer abspaltbaren Schutzgruppen vom Estertyp (wie z.B. Bu$^t$) geschützt vorliegen, in den so erhaltenen Verbindungen zum Schutz von Amino- bzw. Carboxylgruppen eingeführte Schutzgruppen sauer abspaltet und die Verbindungen gegebenenfalls in ihre physiologisch verträglichen Salze überführt.

Schutzgruppen vom Urethantyp sind in Schröder, Lübke, The Peptides, bd. I, New York, London 1965, Seite 22 ff, solche vom Erstertyp ebenda, Seite 52 ff beschrieben.

Man führt des Verfahren vorteilhaft in der Weise durch, daß man

a) eine Verbindung der Formel IIa mit einer Verbindung der Formel IIIa kondensiert,

$R^4\text{-}A^5\text{-OH}$     (IIa)

$H\text{-}A^6\text{-}R^7$     (IIIa)

b) eine Verbindung der Formel IIb mit einer Verbindung der Formel IIIb kondensiert, oder

$R^4\text{-OH}$     (IIb)

$H\text{-}A^5\text{-}A^6\text{-}R^7$     (IIIb)

c) eine Verbindung der Formel IIe mit einer Verbindung der Formel IIIe kondensiert,

$R^1\text{-OH}$     (IIe)

$H\text{-}A^2\text{-}A^3\text{-}A^4\text{-}A^5\text{-}A^6\text{-}R^7$     (IIIe)

wobei die Reste $R^1$ und $R^4$, $R^7$ und $A^2\text{-}A^6$ die im Anspruch 1 definierten Bedeutungen haben, jedoch freie primäre und sekundäre Aminogruppen, die N-terminalen Gruppen der Verbindungen der Formeln IIIa, b und e ausgenommen, mit sauer abspaltbaren Schutzgruppen vom Urethantyp geschützt sind und freie Carboxylgruppen, die C-terminalen Gruppen der Verbindungen der Formeln IIa, b und e ausgenommen, mit sauer abspaltbaren Schutzgruppen vom Erstertyp geschützt sind, anschließend in den nach a) - c) erhaltenen Verbindungen zum Schutz von Amino- bzw. Carboxylgruppen eingeführte Schutzgruppen sauer abspaltet und die erhaltenen Verbindungen gegebenenfalls in ihre physiologisch verträglichen Salze überführt. Die Umsetzung von Verbindungen der Formeln IIb mit Verbindungen der Formel IIIb ist bevorzugt.

3

Die Ausgangsverbindungen der Formeln IIa, b und e und IIIa, b und e sind bekannt oder in an sich bekannter Weise beispielsweise durch Fragmentkondensation zugänglich.

Bei der Synthese der erfindungsgemäßen Peptide kommt als $N^{\alpha}$-Schutzgruppen bevorzugt der Benzyloxycarbonyl- oder 9-Fluorenylmethoxycarbonylrest in Frage, als Carboxylschutzgruppe der tert.-Butylrest.

Die Kondensation nach dem erfindungsgemäßen Verfahren erfolgt nach den allgemeinen Methoden der Peptidchemie, bei Sulfonylverbindungen über das Sulfochlorid, sonst bevorzugt nach der Methode der gemischten Anhydride, über Aktivester, Azide oder nach der Carbodiimid-Methode, insbesondere unter Zusatz reaktionsbeschleunigender und racemisierungsverhindernder Substanzen wie z.B. 1-Hydroxybenzotriazol, N-Hydroxysuccinimid, 3-Hydroxy-4-oxo-3,4-dihydro-1,2,3-benzotriazin, N-Hydroxy-5-norbornen-2,3-dicarboximid, ferner unter Verwendung aktivierter Derivate des 1-Hydroxybenzotriazols oder Anhydriden von Phosphor-, Phosphon- und Phosphinsäuren.

Lösungsmittel sind Dimethylformamid, Dimethylacetamid, Hexamethylphosphorsäuretriamid, N-Methylpyrrolidon oder Dimethylsulfoxid. Sofern die Löslichkeit der Komponenten es erlaubt, können auch Lösungsmittel wie Methylenchlorid oder Chloroform eingesetzt werden. Die genannten Methoden sind z.B. in Meienhofer-Gross: "The Peptides", Academic Press, Vol. I, (1979) beschrieben.

Die Wirkung der erfindungsgemäßen Verbindungen auf das cholinerge System wird nach einer in J. Neurochem. 20 (1973), Seiten 1-8, beschriebenen Methode bestimmt.

Die folgende Tabelle führt einige charakteristische Beispiele auf, welche u.a. die Wirkungssteigerung der basisch substituierten erfindungsgemäßen Peptide gegenüber ACTH und kürzeren, unsubstituierten Peptiden zeigt.

| Nr. | Vergleichssubstanzen: (Verbindung) | Verhalten (Putzen, Gähnen, Strecken) (10 μg i.c.v) | Biochem. Effekte in Striatum Dosis mcg/kg sc; (Ratte) | ACH | CH |
|---|---|---|---|---|---|
| VI. | ACTH-(1-24) | ++ | 0,01-1 | + | + |
| VII | Met-Glu-His-Phe-Arg-Trp-Gly-OH | ∅ | 10-1000 | + | + |
| VIII | Met(0)-Glu-His-Phe-D-Lys-Phe-OH | ∅ | $0,3-1$ | + | + |
| X | Met($O_2$)-Glu-His-Phe-D-Lys-Phe-NH-$(CH_2)_8$-$NH_2$ | ∅ | 0,01 | + | + |
| XI | H-Met($O_2$)-Ala-Ala-Phe-D-Lys-Phe-NH-$(CH_2)_8$-$NH_2$ | | 0,01 | + | + |
| XXII | HOOC-$(CH_2)_5$-CO-Glu-Ala-Phe-D-Lys-Phe-NH-$(CH_3)$-$NH_2$ | | 0,01 | + | + |
| XIII | H-Met($O_2$)-Glu-Ala-Phe-D-Lys-Phe-NH-$(CH_2)_8$-$NH_2$ | | 0,1 | + | + |
| XIV | H-Tyr-Glu-His-Phe-D-Lys-Phe-NH-$(CH_2)_8$-$NH_2$ | + | 0,1 | + | + |
| | Z-D-Lys-Phe-NH-$(CH_2)_8$-$NH_2$ | | 1,0 | + | + |
| XVI | pyro-Glu-Glu-His-Phe-D-Lys-Phe-NH-$(CH_2)_8$-$NH_2$ | | 0,1 | + | + |
| XIII | H-Met(0)-Glu-His-Phe-D-Lys-Phe-NH-$(CH_2)_6$-$NH_2$ | | 0,01 | + | + |

ACH = Acetylcholin

CH = Cholin

Die erfindungsgemäßen Verbindungen bewirken bei Mäusen eine signifikante, dosisabhängige Abschwächung der durch Elektrokrampf oder Scopolamin hervorgerufenen Amnesie ("one-trial passive avoidance test").

Beim Menschen wirken die erfindungsgemäßen Peptide stimmungsaufhellend, antidepressiv und anxiolytisch. Sie erhöhen die Aufmerksamkeit gegenüber der Umwelt, verbessern die Lern- und Gedächtnisleistung, wirken sich günstig bei Resozialisierungsprozessen aus und können bei allen Erkrankungen, posttraumatischen und degenerativen Hirnschäden angewandt werden, die mit verminderter Funktion des zentralen Acetylcholinstoffwechsels verbunden sind, z.B. milder Dementia und auch Frühformen der Alzheimer'schen Krankheit etc. Weiterhin vermindern sie die Hyperemotionalität.

Als Arzneimittel werden die erfindungsgemäßen Verbindungen, wenn sie nicht als Zwitterionen vorliegen, in Form ihrer Salze mit physiologisch unbedenklichen Säuren wie z.B. Essigsäure, Malonsäure, Zitronensäure, Apfelsäure, aber auch als Hydrochlorid oder Sulfat angewandt. Bei einem normalgewichtigen Erwachsenen wird die intranasale Applikation vorzugsweise in einer Dosierung von 0,1 $\mu$g bis 1 mg pro Dosis angewandt, besonders bevorzugt sind 1 bis 500, insbesondere 5 bis 200 $\mu$g pro Dosis. Die erfindungsgemäßen Arzneimittel können beispielsweise bis zu 6 mal, vorzugsweise bis zu 3 mal pro Tag appliziert werden. In vielen Fällen genügt auch die Applikation einer Dosis pro Tag. Die erfindungsgemäßen Verbindungen können auch subcutan mit 0,001 bis 10 $\mu$g/kg, vorzugsweise 0,01 bis 5 $\mu$g/kg, insbesondere 0,05 bis 2 $\mu$g/kg verabreicht werden. In Abhängigkeit von der Konstitution sind sie mehr oder weniger peroral resorbierbar. Der Dosisspielraum bei peroraler Gabe ist vergleichsweise groß und liegt zwischen 0,1 und 50 mg täglich, auf mehrere Verabreichungen verteilt. Bevorzugte Einzeldosis ist bei den am stärksten wirkenden Verbindungen 0,1 bis 10 mg.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige alkoholische oder ölige Suspensionen oder wäßrige alkoholische Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungvermittler, Emulgatoren oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Beispiele

Bei den folgenden Herstellungsbeispielen werden die in der Peptidchemie eingeführten Abkürzungen verwendet. Weitere, häufig gebrauchte Abkürzungen sind:

| | |
|---|---|
| DMF | Dimethylformaid |
| NEM | N-Ethylmorpholin |
| DCC | Dicyclohexylcarbodiimid |
| DCH | Dicyclohexylharnstoff |
| DCA | Dicyclohexylamin |
| HOBt | 1-Hydroxybenzotriazol |
| HOObt | 3-Hydroxy-4-oxo-3,4-dihydro-1,2,3-benzotriazin |
| Z | Benzyloxycarbonyl |
| Boc | tert-Butyloxycarbonyl |
| $Bu^t$ | tert.-Butyl |
| Me | Methyl |
| Et | Ethyl |
| Tic | 1,2,3,4-Tetrahydroisochinolin-3-carbonsäure (L-Form) |
| HONp | 4-Nitrophenol |
| HOTcp | 2,4,5-Trichlorphenol |
| HONSu | N-Hydroxysuccinimid |
| DC | Dünnschichtchromatographie/Dünnschichtchromatogramm in den Laufmitteln: |
| A : | Methylethylketon:Pyridin:Wasser:Essigsäure (70:15:15:2) |
| B: | n-Butanol:Essigsäure:Wasser (6:2:2) |
| C: | n-Butanol:Pyridin:Essigsäure:Wasser (4:1:1:5); davon die Oberphase |
| D: | Heptan:tert-Butanol:Pyridin (3:1:1) |

Beispiel 1

Z-D-Lys-Phe-NH-$(CH_2)_8$-NH$_2$ $\cdot$ 2 HCl

6

a) Boc-NH-$(CH_2)_8$-NH-Boc

65 g 1,8-Diaminooctan werden in 800 ml Dioxan und 400 ml Wasser gelöst. Man gibt 69 ml Triethylamin und anschließend unter Vibromischung portionsweise unterhalb 20°C 216 g Di-tert.butyl-dicarbonat zu. Dabei fällt das Reaktionsprodukt bereits aus. Man rührt 2 Stunden nach, filtriert den Niederschlag ab, der mit wenig Wasser gewaschen und getrocknet wird. Das Filtrat wird im Vakuum eingeengt, wobei mehrmals ein Niederschlag abgesaugt wird, den man wie oben behandelt. Die Niederschläge werden gesammelt, mit Wasser digeriert und getrocknet. Ausbeute: 118 g Schmp. 103-104°C

| $C_{18}H_{36}N_2O_4$ (344.4) | Ber. C 62,75 | H 10,53 | N 6,97 |
| | gef. C 63,0 | H 10,7 | N 7,2 |

b) Boc-NH-$(CH_2)_8$-$NH_2$ . HCl

68 g der nach a) hergestellten Verbindung werden in 1 l trockenem Ether suspendiert, der 2N HCl enthält. Man rührt 3 Stunden bei Raumtemperatur, kühlt auf etwa 0°C, filtriert den Niederschlag ab und wäscht ihn mit trockenem Ether.
Ausbeute: 38 g, Schmp: 150 - 152°C (Zers.)

| $C_{13}H_{29}ClN_2O_2$ (280,8) | Ber. C 55,65 | H 10,4 | N 10,0 | Cl:12,6 |
| | gef. C 55,6 | H 10,5 | N 10,2 | Cl:12,5 |

c) Z-D-Lys(Boc)-Phe-OMe

43,2 g Z-D-Lys(Boc)-OH, hergestellt analog der L-Verbindung, werden in 400 ml Dimethylformamid gelöst. Man gibt 24,5 g H-Phe-OMe•HCl, 15,33 g HOBt, 14,53 ml NEM und unter Rühren 25 g DCC zu und läßt über Nacht bei Raumtemperatur stehen. Nach Abfiltrieren des Harnstoffs destilliert man das Lösungsmittel im Vakuum ab und kristallisiert den öligen Rückstand aus 200 ml 80-proz. Ethanol um.
Ausbeute 57.9 g (94 % d.Th.)
Zur Analyse wird eine Probe aus DMF mit Ether/Petrolether (1:1) gefällt und nochmals aus 80-proz. Ethanol kristallisiert.

| $C_{29}H_{39}N_3O_7$ (541,6) | Ber. C 64,31 | H 7,26 | N 7,76 |
| | gef. C 64,3 | H 7,3 | N 7,5 |

$[\alpha]_D^{20}$ : + 5.5° (c = 0,5 in 90-proz. Essigsäure)

d) Z-D-Lys(Boc)-Phe-OH

56,0 g des Methylesters werden in einer Mischung aus 500 ml Dioxan, 200 ml Methanol und 60 ml Wasser in der Wärme gelöst und bei pH 12,5 mit 1N NaOH innerhalb 150 min. verseift. Man bringt mit 2N HCl unter Rühren auf pH 7, destilliert die organischen Lösungsmittel ungeachtet des Niederschlags im Vakuum weitgehend ab, gibt 800 ml eiskalten Essigester zu und versetzt unter Eiskühlung und kräftigem Rühren vorsichtig mit 35 ml 2N HCl. Die Schichten werden getrennt, die Essigesterlösung mit etwas Wasser gewascher, über Natriumsulfat getrocknet und im Vakuum eingeengt. Der zunächst ölige Rückstand kristallisiert nach einiger Zeit durch, die Kristalle werden mit Petrolether gewaschen und im Vakuum getrocknet.
Ausbeute 50,2 g (92 %). Schmp. 85-87°C.

| $C_{28}H_{37}N_3O_7$ (527,6) | Ber. C 63,74 | H 7,09 | N 7,96 |
| | gef. C 63,3 | H 7,3 | N 8,4 |

$[\alpha]_D^{20}$ : +10,7° (c = 1 in 90-proz. Essigsäure)

e) Z-D-Lys(Boc)-Phe-NH-$(CH_2)_8$-NH-Boc

48,5 g des nach d) hergestellten Dipeptids und 25,8 g des nach b) hergestellten Boc-Diamin-hydrochlorids werden in 1 l DMF gelöst. Man gibt nacheinander 14,8 g HOObt, 15 ml NEM und unter Kühlen 22,5 g DCC zu, rührt 1 h und läßt über Nacht bei Raumtemperatur stehen. Man filtriert vom Harnstoff ab, destilliert das Lösungsmittel im Vakuum ab und kristallisiert den Rückstand aus 300 ml 80-proz. Ethanol um. Der Niederschlag läßt sich nur schwierig abfiltrieren und wird vorteilhaft durch Zentrifugieren gewonnen.
Ausbeute 60,2 g (86,9 %).

| $C_{41}H_{62}N_5O_8$ (753,0) | Ber. C 65,40 | H 8,30 | N 9,3 |
|---|---|---|---|
| | Gef. C 65,6 | H 8,5 | N 9,3 |

$[\alpha]_D^{20}$ : + 2,0° (c = 1 in 90-proz. Essigsäure)

f) Z-D-Lys-Phe-NH-$(CH_2)_8$-$NH_2$ . 2HCl

753 mg der nach e) erhaltenen Verbindung werden in 7,5 ml konz. HCl/Wasser/Ameisensäure (0,6:0,6:9,6) 30 min stehen gelassen. Man destilliert das Lösungsmittel im Vakuum ab und destilliert mit Toluol nach. Der Rückstand verfestigt sich beim Stehen unter Ether, wird abfiltriert, mit Ether gewaschen und im Vakuum über KOH getrocknet.
Ausbeute 650 mg.
Die Verbindung ist im DC auf Silicagel im Laufmittel A einheitlich.
Elementaranalyse im Rahmen der Fehlergrenze.

Beispiel 7

Z-D-Lys-Phe-D-Lys-Phe-Gly-NH-$(CH_2)_4$-$NH_2$ . 3HCl

a) Z-D-Lys(Boc)-Phe-Gly-OMe

5,3 g Z-D-Lys(Boc)-Phe-OH (Beispiel 1d) werden in 60 ml DMF nacheinander unter Rühren mit 1,26 g G-Gly-OMe . HCl, 1,6 g HCObt, 1,3 ml NEM und 2,2 g DCC versetzt. Nach Stehen über Nacht wird vom Harnstoff abfiltriert, das Filtrat im Vakuum eingeengt. Der Rückstand wird in 200 ml Essigester aufgenommen, die Lösung in der Kälte mit 10-proz. Citronensäurelösung, gesättigt. Natriumhydrogencarbonatlösung und Wasser gewaschen, im Vakuum auf ca. 40 ml eingeengt und mit Ether/Petrolether 1:1 versetzt. Der Niederschlag wird abfiltriert und mit Ether/Petrolether 1:1 gewaschen und getrocknet.

b) H-D-Lys(Boc)-Phe-Gly-OMe, TosOH

4,21 g der nach a) erhaltenen Verbindung werden in 70 ml MeOH unter Titration mit 1N methanol. TosOH bei pH 4,5 an Pd katalytisch hydriert. Nach Abfiltrieren des Katalysators wird das Lösungsmittel im Vakuum abdestilliert, der Rückstand mehrmals mit Diisopropylether verrieben und getrocknet.
Ausbeute 3,82 g, einheitlich im DC(A,C).

c) Z-D-Lys(Boc)-Phe-D-Lys(Boc)-Phe-Gly-OMe

5,3 g Z-D-Lys(Boc)-Phe-OH und 6,4 g H-D-Lys(Boc)-Phe-Gly-OMe, TosOH werden in 100 ml DMF unter Rühren mit 1,6 g HOObt, 1,3 g NEM und 2,2 g DCC versetzt. Nach Stehen über Nacht und Abfiltrieren des Harnstoffs wird das Lösungsmittel im Vakuum abdestilliert. Der Rückstand wird in 300 ml Essigester/n-Butanol (2:1) aufgenommen und wie oben mit Citronensäurelösung, Natriumhydrogencarbonatlösung und Wasser gewaschen, über Natriumsulfat getrocknet, das Lösungsmittel im Vakuum abdestilliert. Der Rückstand wird aus 60 ml Isopropanol umkristallisiert. Ausbeute 7,0 g, einheitlich in DC(A).

d) Z-D-Lys(Boc)-Phe-D-Lys(Boc)-Phe-Gly-OH

5,4 g des Methylesters werden in 50 ml Dioxan + 50 ml Methanol + 20 ml Wasser warm gelöst und bei pH 13 mit 1N NaOH verseift. Man bringt mit 2N HCl in der Kälte auf pH 6, destilliert den größten Teil des Lösungsmittels ab, versetzt mit 100 ml Essigester und 20 ml n-Butanol, säuert in der Kälte mit 1N HCl bis pH 2 an und wäscht mit Wasser. Die organische Phase wird über Natriumsulfat getrocknet und im Vakuum eingedampft. Der ölige Rückstand wird beim Verreiben mit Ether fest.
Ausbeute 5,1 g, nahezu einheitlich im DC (A).

e) Z-D-Lys(Boc)-Phe-D-Lys(Boc)-Phe-Gly-NH-$(CH_2)_4$-NH-Boc

1,9 g der nach d) erhaltenen Verbindung werden in 30 ml DMF mit 0,44 g Boc-NH-$(CH_2)_4$-$NH_2$ • HCl, hergestellt nach Liebig's Ann. Chem. 750 (1971) 165, und unter Rühren weiter mit 0.27 g HOBt, 0,3 ml NEM und 0,45 g DCC versetzt. Nach Stehen über Nacht, Abfiltrieren des Harnstoffs und Abdestillieren von DMF im Vakuum wird der Rückstand in 60 ml Essigester/n-Butanol (1:1) aufgenommen und wie oben mit Citronensäurelösung, Hydrogencarbonatlösung und Wasser gewaschen, getrocknet und im Vakuum vom Lösungsmittel befreit. Der Rückstand wird aus Isopropanol umkristallisiert.
Ausbeute 1,55 g, einheitlich in DC (A,C).

f) Z-D-Lys-Phe-D-Lys-Phe-Gly-NH-$(CH_2)_4$-$NH_2$ . 3HCl

582 mg werden analog Beispiel 1f in 5,4 ml HCl/HCCOH gelöst und wie dort aufgearbeitet.
Ausbeute 440 mg, einheitlich in DC (B,C).

Beispiel 8

H-Phe-D-Lys-Phe-NH-$(CH_2)_8$-$NH_2$ . 3HCl

a) Z-Phe-D-Lys(Boc)-Phe-NH-$(CH_2)_8$-NH-Boc

10,8 g H-D-Lys(Boc)-Phe-NH-$(CH_2)_8$-NH-Boc . TosOH, hergestellt aus der Z-Verbindung analog Beispiel 7b durch katalytische Hydrierung in Methanol bei pH 4,5 unter Titration mit 1N TosOH, werden in 100 ml DMF gelöst und unter Rühren nacheinander mit 4,1 g Z-Phe-OH, 1,8 g HOBt, 2 ml NEM und 3,0 g DCC versetzt. Nach Stehen über Nacht wird von Harnstoff abfiltriert und DMF im Vakuum abdestilliert. Den Rückstand kristallisiert man aus 50 ml 80-proz. Ethanol um.
Ausbeute 8,2 g, einheitlich in DC(A).

| $C_{50}H_{71}N_6O_9$ (900,2) | Ber. C 66,72 | H 7,95 | N 9,34 |
| | Gef. C 66,7 | H 7,9 | N 9,1 |

$[\alpha]_D^{20}$ : + 3,0° (c = 1 in 90-proz. Essigsäure)

b) H-Phe-D-Lys(Boc)-Phe-NH-$(CH_2)_8$-$NH_2$ . HCl

1,0 g der nach a) erhaltenen Verbindung werden analog Beispiel 7b katalytisch hydriert, wobei das pH jedoch mit 1N HCl in Methanol aufrechterhalten wird. Nach analoger Aufarbeitung erhält man 0,92 g, einheitlich in DC(D)

c) H-Phe-D-Lys-Phe-NH-$(CH_2)_8$-$NH_2$ . 3HCl

Abspaltung der Boc-Schutzgruppen von der nach b) erhaltenen Verbindung wie in Beispiel 1f.
Ausbeute 0,86 g, nahezu einheitlich in DC (A,B).
Aminosäureanalyse: Phe:Lys (2,0 : 1,04)

Beispiel 9

Z-Glu-His-Phe-D-Lys-Phe-NH-$(CH_2)_8$-$NH_2$ . 2 HCl

a) H-Phe-D-Lys(Boc)-Phe-NH-$(CH_2)_8$-NH-Boc . TosOH

7,5 g der nach Beispiel 8a hergestellten Z-Verbindung werden analog Beispiel 7b in Methanol katalytisch hydriert und aufgearbeitet.
Ausbeute 7,3 g, einheitlich in DC(D).

b) Z-Glu(OBu$^t$)-His-Phe-D-Lys(Boc)-Phe-NH-(CH$_2$)$_8$-NH-Boc

Man setzt 5,16 g der nach a) erhaltenen Verbindung in 70 ml DMF mit 2,76 g Z-Glu(OBu$^t$)-His-OH, 0,9 g HOObt, 0,7 ml NEM und 1,22 g DCC um und arbeitet analog Beispiel 4a auf. Der nach Abdestillieren des Essigesters verbleibende Rückstand wird mehrmals mit Ether/Petrolether (1:1) verrieben und getrocknet.
Ausbeute 5,0 g, Lt. DC(A,C) nur mit sehr wenig DCH verunreinigt.

c) Z-Glu-His-Phe-D-Lys-Phe-NH-(CH$_2$)$_8$-NH$_2$ . 2 HCl

Man spaltet aus 0,5 g der nach b) erhaltenen Verbindung analog Beispiel 1f Boc- und Bu$^t$-Schutzgruppen ab und arbeitet wie dort beschrieben auf. Dann wird nochmals in wenig Wasser gelöst, mit wenig schwach basischem Ionenaustauscher bis pH 4,5 gerührt, filtriert und im Vakuum zur Trockene eingeengt. Der Rückstand wird mit Ether verrieben und getrocknet.
Ausbeute 0,3 g, nahezu einheitlich in DC(A,B). Aminosäureanalyse korrekt.

Beispiel 11

H-Met(O)-Glu-His-Phe-D-Lys-Phe-NH-(CH$_2$)$_8$-NH$_2$ . 3 CH$_3$COOH

a) Boc-Met-Glu(OBu$^t$)-His-Phe-D-Lys(Boc)-Phe-NH-(CH$_2$)$_8$-NH-Boc

Man stellt aus 3,75 g der nach Beispiel 9b erhaltenen Verbindung analog Beispiel 7b 4,2 g H-Glu(OBu$^t$)-His-Phe-D-Lys(Boc)-Phe-NH-(CH$_2$)$_8$-NH-Boc•TosOH her. Hiervon setzt man 2,56 g in 60 ml DMF mit 1,2 g Boc-Met-ONp, 27 mg HOBt und 0,4 ml NEM über Nacht bei Raumtemperatur um, destilliert das Lösungsmittel im Vakuum ab und fällt den Rückstand nach Digerieren mit NaHCO$_3$ und Wasser aus Essigester/Ether um, digeriert mit Ether und trocknet.
Ausbeute 2,2 g, einheitlich im DC(C).

b) Boc-Met(O)-Glu(OBu$^t$)-His-Phe-D-Lys(Boc)-Phe-NH-(CH$_2$)$_8$-NH-Boc

1,8 g der nach a) erhaltenen Verbindung werden in 36 ml Essigsäure mit 2 ml 3-proz. H$_2$O$_2$ oxidiert. Nach 30 min wird im Vakuum eingeengt und der Rückstand mit Wasser und Ether digeriert.

c) H-Met(O)-Glu-His-Phe-D-Lys-Phe-NH-(CH$_2$)$_8$-NH$_2$ .3CH$_3$COOH

Das nach b) erhaltene Rohprodukt wird mit 25 ml HCl/HCOOH analog Beispiel 1f von den Schutzgruppen befreit. Man dampft im Vakuum zur Trockene ein, löst den Rückstand in 30 ml 50-proz. Methanol. Behandelt mit Ionenaustauscher Amberlite® IRA 93 in der Acetatform, bis der pH-Wert auf etwa 4,2 zurückgegangen ist, filtriert und bringt die Lösung im Vakuum zur Trockene.
Ausbeute 1,36 g.
Zur Reinigung löst man in 6 ml 1-proz. Essigsäure und chromatographiert in demselben Lösungsmittel über eine Säule 200 x 2,5 cm Sephadex® LH-20. In den Fraktionen 5 und 6 findet man 480 mg chromatographisch einheitliches Peptid mit korrekter Aminosäureanalyse.
$[\alpha]_D^{20}$ : + 4.9° (c = 0,5 in 90-proz. Essigsäure)
In Fraktion 7 findet man 290 mg eines Gemisches mit dem stereoisomeren Met(O)-Derivat.

Beispiel 13

pGlu-Glu-His-Phe-D-Lys-Phe-NH-(CH$_2$)$_8$-NH$_2$ . 2 CH$_3$COOH

a) pGlu-Glu(OBu$^t$)-His-Phe-D-Lys(Boc)-Phe-NH-(CH$_2$)$_8$-NH-Boc

1,6 g H-Glu(OBu$^t$)-His-Phe-D-Lys-Phe-NH-(CH$_2$)$_8$-NH$_2$-Boc . TosOH (Beispiel 11a) und 370 mg pGlu-OTcp werden in 20ml DMF in Anwesenheit von 0,17 ml NEM und 17 mg HOBt über Nacht zur Reaktion gebracht. Man destilliert das Lösungsmittel im Vakuum ab und digeriert den Rückstand mit Ether. Ausbeute 1,6 g

b) Das unter a) erhaltene Rohprodukt wird analog Beispiel 1f von den Schutzgruppen befreit und analog Beispiel 11c chromatographisch gereinigt. Man erhält 465 mg der im DC(C) einheitlichen Titelverbindung. Aminosäureanalyse korrekt.

Beispiel 14

Z-Tyr-Glu-His-Phe-D-Lys-Phe-NH-$(CH_2)_8$-$NH_2$ . 2 $CH_3COOH$

1,28 g des in Beispiel 11a beschriebenen teilgeschützten Pentapeptid-amids werden in 20 ml DMF mit 610 mg Z-Tyr(Bu$^t$)-ONSu in Anwesenheit von 13,5 mg HOBt und 0,13 ml NEM umgesetzt. Nach Stehen über Nacht wird das Lösungsmittel im Vakuum abdestilliert. Der Rückstand wird nach Verreiben mit Ether analog Beispiel 1f mit HCl/HCOOH behandelt. Nach Abdestillieren des Lösungsmittels wird analog Beispiel 11c chromatographiert.
Ausbeute an chromatographisch einheitlichem Peptid 360mg. Aminosäureanalyse korrekt.

Beispiel 15

H-Tyr-Glu-His-Phe-D-Lys-Phe-NH-$(CH_2)_8$-$NH_2$ . 3 $CH_3COOH$

Man verfährt nach Beispiel 14, setzt jedoch 565 mg Boc-Tyr(Bu$^t$)-ONSu ein und erhält 280 mg der Titelverbindung.
Einheitlich im DC(C). Aminosäureanalyse korrekt.

Beispiel 16

H-Met($O_2$)-Glu-His-Phe-D-Lys-Phe-NH-$(CH_2)_8$-$NH_2$ . 3HCl

a) Boc-Met($O_2$)-Glu(OBu$^t$)-His-Phe-D-Lys(Boc)-Phe-NH-$(CH_2)_8$-NH-Boc

370 mg Boc-Met($O_2$)-OH und 1,4 g H-Glu(OBu$^t$)-His-Phe-D-Lys-(Boc)-Phe-NH-$(CH_2)_8$-NH-Boc•TosOH werden in 25 ml DMF mit 0,14 ml NEM und 150mg HOBt versetzt. Man gibt 242 mg DCC und nach 30 Min. nochmals 0.07 ml NEM zu und arbeitet nach Stehen über Nacht auf. Das Rohprodukt wird in feuchtem Essigester gelöst, nacheinander mit 10 % $KHSO_4$/$K_2SO_4$-Lösung, gesättigem Natriumhydrogencarbonatlösung und Wasser gewascher, die Essigesterlösung kurz über Natriumsulfat getrocknet, auf ein kleines Volumen eingeengt und unter kräftigem Rühren in Ether getropft.
Ausbeute an unlöslichem Rohprodukt 1,1 g.

b) H-Met($O_2$)-Glu-His-Phe-D-Lys-Phe-NH-$(CH_2)_8$-$NH_2$ . 3$CH_3COOH$

650g der nach a) erhaltenen Verbindung werden analog Beispiel 11 von den Schutzgruppen befreit und aufgearbeitet.
Ausbeute 520 mg
350 mg werden in 2 ml 1N Essigsäure gelöst und an Sephadex® LH 20 (2,5 x 100 cm) chromatogrpahiert. Die im DC einheitlichen Hauptfraktionen werden zusammengefaßt und lyophilisiert.
Ausbeute 230 mg einheitlich im DC (C). Aminosäureanalyse korrekt.

Beispiel 17

Z-Glu-Ala-Phe-D-Lys-Phe-NH-$(CH_2)_8$-$NH_2$ . 2 HCl

a) Z-Ala-Phe-D-Lys(Boc)-Phe-NH-$(CH_2)_8$-NH-Boc

20,85 g teilgeschütztes Tripeptid (Beispiel 8b) und 4,95 g Z-Ala-OH werden in 300 ml DMF unter Rühren mit 12,7 g HOBt, 2,84 g NEM und 4,88 g DCC versetzt. Nach Stehen über Nacht wird von DCH abfiltriert und das Lösungsmittel im Vakuum abdestilliert. Man fällt zweimal aus Ethanol/Wasser und trocknet im Vakuum.

Ausbeute 18,5 g. $[\alpha]_D^{20}$ : -11° (c = 1,in 90-proz. Essigsäure)

| $C_{53}H_{77}N_7O_{10}$ (972,3) | Ber. C 65,47 | H 7,98 | N 10,09 |
|---|---|---|---|
| | Gef. C 65,6 | H 8,0 | N 10,3 |

b) H-Ala-Phe-D-Lys(Boc)-Phe-NH-(CH$_2$)$_8$-NH-Boc . TosOH

16 g der nach a) hergestellten Z-Verbindung werden in Methanol/DMF unter Zugabe von 1N TosOH bei pH 6 an Pd katalytisch hydriert. Man filtriert vom Katalysator ab, klärt mit Aktivkohle, destilliert das Lösungsmittel ab und verreibt den Rückstand mit Essigester.
Ausbeute nach Trocknen 12,4 g

| $C_{55}H_{84}N_8O_{12}$ (1081,35) | Ber. C 61,09 | H 7,83 | N 10,36 | S 2,9 |
|---|---|---|---|---|
| | Gef. C 60,9 | H 7,8 | N 10,2 | S 3,2 |

c) Z-Glu-(OBu$^t$)-Ala-Phe-D-Lys(Boc)-Phe-NH-(CH$_2$)$_8$-NH-Boc

5.0 g der nach b) erhaltenen Verbindung werden in 50 ml DMF mit 2,03 g Z-Glu(OBu$^t$)-OH und danach unter Rühren mit 675 mg HOBt, 0,71 ml NEM und 1,13 g DCC versetzt. Nach Stehen über Nacht wird DCH abfiltriert, das Lösungsmittel abdestilliert, der Rückstand in n-Butanol/Essigester (1:1) aufgenommen, mit Natriumhydrogencarbonatlösung und Wasser gewaschen und über Na$_2$SO$_4$ getrocknet. Der nach Abdestillieren des Lösungsmittels verbleibende Rückstand wird mit Ether digeriert und getrocknet.
Ausbeute 5,24 g, einheitlich im DC(D).

d) Z-Glu-Ala-Phe-D-Lys-Phe-NH-(CH$_2$)$_8$-NH$_2$ . 2 HCl

300 mg der nach c) erhaltenen Verbindung werden zur Abspaltung der Schutzgruppen wie in Beispiel 1f behandelt. Der Rückstand wird nach Aufarbeitung mit Ether verrieben und getrocknet.
Ausbeute 160 mg.
Einheitlich in DC(D) . $[\alpha]_D^{20}$ : -15° (c = 1, in MeOH) Aminosäureanalyse korrekt.

Beispiel 19

H-Met(O$_2$)-Glu-Ala-Phe-D-Lys-Phe-NH-(CH$_2$)$_8$-NH$_2$ . 3HCL

a) Boc-Met(O$_2$)-Glu(OBu$^t$)-Ala-Phe-D-Lys(Boc)-Phe-NH-(CH$_2$)$_8$-NH-Boc

1,2 g des nach Beispiel 21 a hergestelltenH-Gln(OBu$^t$)-Ala-Phe-D-Lys(Boc)-Phe-NH-(CH$_2$)$_8$-NH-Boc . TosOH, werden in DMF mit 344 mg Boc-Met(O$_2$)-OH, 137 mg HOBt, 0,13 ml NEM und 225 mg DCC versetzt und nach anfänglichem Rühren 15 Stunden stehen gelassen. Man filtriert, destilliert das Lösungsmittel im Vakuum ab und verreibt den Rückstand mit Wasser und Ether.
Ausbeute 1,2 g. Die Verbindung enthält noch wenig DCH, sonst einheitlich im DC(A,D).

b) H-Met(O$_2$)-Glu-Ala-Phe-D-Lys-Phe-NH-(CH$_2$)$_8$-NH$_2$ . 3HCl

Die nach a) hergestellte Verbindung wird analog Beispiel 1f mit Ameisensäure/HCl behandelt und aufgearbeitet. Dann wird nochmals in Waser gelöst, filtriert und im Vakuum zur Trockene gebracht. Der Rückstand wird in Essigester verrieben und getrocknet.
Ausbeute aus 1,0 g: 0,78 g. Einheitlich im DC(A,B,C).
$[\alpha]_D^{20}$ : -12,5° (c = 1 ,Methanol) Aminosäureanalyse korrekt.

Beispiel 21

Glutaroyl-Glu-Ala-Phe-D-Lys-Phe-NH-(CH$_2$)$_8$-NH$_2$ . 2 HCl

a) Glutaroyl-Glu(OBu$^t$)-Ala-Phe-D-Lys(Boc)-Phe-NH-(CH$_2$)-NH-Boc

1,2 g H-Glu(OBu$^t$)-Ala-Phe-D-Lys(Boc)-Phe-NH-(CH$_2$)$_8$-NH-Boc . TosOH, hergestellt durch katalytische Hydrierung der nach Beispiel 17c erhaltenen Z-Verbindung, werden in 10 ml Pyridin gelöst. Man gibt 140 mg Glutarsäure-anhydrid zu und läßt 15 Stunden bei Raumtemperatur reagieren. Der nach Einengen im Vakuum verbleibende Rückstand wird mit Essigester verrieben, das feste Produkt wird abfiltriert, mit Ether gewaschen und getrocknet.
Ausbeute 1,08 g. Einheitlich im DC(A,B,C)

b) Glutaroyl-Glu-Ala-Phe-D-Lys-Phe-NH-(CH$_2$)$_8$-NH$_2$ . 2HCl

0.9 g der nach a) erhaltenen Verbindung werden analog Beispiel 1f mit Ameisensäure/HCl behandelt.
Ausbeute 0,6 g.
$[\alpha]_D^{20}$ : -19,6° (c = 1, Methanol)
Aminosäure- und Elementaranalyse korrekt.
Durch Lösen in Wasser, Behandeln mit schwach basischen Ionenaustauschern bis pH 5-6 und Einengen der filtrierten Lösung erhält man die Titelverbindung.

Beispiel 22

Z-Ala-Ala-Phe-D-Lys-Phe-NH-(CH$_2$)$_8$-NH$_2$ . 2 HCl

a) Z-Ala-Ala-Phe-D-Lys(Boc)-Phe-NH-(CH$_2$)$_8$-NH-Boc

20,85 g teilgeschütztes Tripeptid (Beispiel 8b) werden in 200 ml DMF mit 6,53 g Z-Ala-Ala-OH, 12,66 g HOBt, 2,84 ml NEM und 4,9 g DCC umgesetzt und analog Beispiel 4a aufgearbeitet.
Ausbeute 16,7 g, einheitlich in DC(C,D).
$[\alpha]_D^{20}$ : -12,0° (c = 1, in 90-proz. Essigsäure)

| C$_{56}$H$_{82}$N$_8$O$_{11}$ (1043,3) | Ber. C 64,47 | H 7,92 | N 10,74 |
|---|---|---|---|
| | Gef. C 64,2 | H 8,0 | N 10,6 |

b) Z-Ala-Ala-Phe-D-Lys-Phe-NH-(CH$_2$)$_8$-NH$_2$ . 2 HCl

500 mg der nach a) erhaltenen Verbindung werden analog Beispiel 1f von den Schutzgruppen befreit. Die Verbindung ist bereits ohne chromatographische Reinigung in DC(D) einheitlich.
$[\alpha]_D^{20}$ : -15,2° (c = 1, in 90-proz. Essigsäure)

| C$_{46}$H$_{68}$Cl$_2$N$_8$O$_7$ (916,0) | Ber.C 60,32 | H 7,98 | N 12,23 | Cl 7,74 |
|---|---|---|---|---|
| | Gef.C 60,1 | H 7,9 | N 12,1 | Cl 7,9 |

Aminosäureanalyse korrekt.

Beispiel 23

H-Gly-Ala-Ala-Phe-D-Lys-Phe-NH-(CH$_2$)$_8$-NH$_2$ . 3 HCl

a) H-Ala-Ala-Phe-D-Lys(Boc)-Phe-NH-(CH$_2$)$_8$-NH-Boc . TosOH

16 g der nach Beispiel 22a erhaltenen Verbindung werden analog Beispiel 17b katalytisch hydriert und aufgearbeitet.
Ausbeute 12,9 g.

| $C_{55}H_{84}N_8O_{12}S$ (1081,35) | Ber.C 61,09 | H 7,83 | N 10,36 | S 2,96 |
|---|---|---|---|---|
| | Gef.C 60,9 | H 7,8 | N 10,2 | S 3,2 |

b) Boc-Gly-Ala-Ala-Phe-D-Lys(Boc)-Phe-NH-$(CH_2)_8$-NH-Boc

973 mg der nach a) erhaltenen Verbindung werden in 10 ml DMF mit 260 mg Boc-Gly-ONSu umgesetzt, die Titelverbindung nach Abdestillieren des Lösungsmittel im Vakuum durch Verreiben mit Essigester isoliert.
Ausbeute 850 mg, einheitlich in DC(D)

| $C_{55}H_{87}N_9O_{12}$ (1066,37) | Ber.C 61,95 | H 8,22 | N 11,82 |
|---|---|---|---|
| | Gef.C. 61,8 | H 8,3 | N 11,6 |

c) H-Gly-Ala-Ala-Phe-D-Lys-Phe-NH-$(CH_2)_8$-$NH_2$ . 3HCl

800 mg der nach b) erhaltenen Verbindung werden analog Beispiel 1f von den Schutzgruppen befreit.
Ausbeute 610 mg, einheitlich in DC(B,D).
$[\alpha]_D^{20}$ : -24,0° (c = 1, in Methanol)

| $C_{40}H_{66}Cl_3N_9O_6$ (875,34) | Ber.C 54,88 | H 7,60 | N 14,4 | Cl 12,5 |
|---|---|---|---|---|
| | Gef.C 55,0 | H 7,5 | N 14,2 | Cl 12,8 |

Aminosäureanalyse korrekt.

Beispiel 24

H-Tyr-Ala-Ala-Phe-D-Lys-Phe-NH-$(CH_2)_8$-$NH_2$ . 3 HCl

0,97 g der nach Beispiel 17 a erhaltenen Verbindung werden in 10 ml DMF mit 435 mg Boc-Tyr(Bu$^t$)-ONSu unter Zugabe von 0,13 ml NEM umgesetzt. Aufarbeitung nach Beispiel 13a. Das Produkt (0,8 g) wird anschließend analog Beispiel 1f von den Schutzgruppen befreit.
Ausbeute 0,65 g
$[\alpha]_D^{20}$ : -15,0° (c = 1, Methanol).
Einheitlich im DC(A,B,C).
Aminosäureanalyse korrekt.

Beispiel 25

Glutaroyl-Ala-Ala-Phe-D-Lys-Phe-NH-$(CH_2)_8$-$NH_2$ . 2 HCl

Man setzt das nach Beispiel 23a erhaltene teilgeschützte Peptid analog Beispiel 21a mit Glutarsäure-anhydrid um, spaltet die Schutzgruppen ab und arbeitet wie beschrieben auf.
$[\alpha]_D^{20}$ : -19,6° (c = 1, Methanol).
Aminosäureanalyse korrekt. Durch Lösen in Wasser, behandeln mit schwach basischem Ionenaustauscher bis pH 5-6 erhält man das Mono-Hydrochlorid, das durch Einengen der Lösung und Verreiben des Rückstandes mit Ether isoliert wird.

Beispiel 26

H-Met($O_2$)-Ala-Ala-Phe-D-Lys-Phe-NH-$(CH_2)_8$-$NH_2$ . 3 HCl

Analog Beispiel 16 erhält man aus dem nach Beispiel 23a hergestellten Peptid und Abspalten der Schutzgruppen die Titelverbindung.

$[\alpha]_D^{20}$ : -15,3° (c = 1, Methanol).
Aminosäureanalyse korrekt. Einheitlich im DC(A,B,C).

Beispiel 40

Z-D-Lys-Phe-NH-$(CH_2)_9$-NH$_2$ . 2 HCl

a) Z-D-Lys(Boc)-Phe-NH-$(CH_2)_9$-NH-Boc

27 g NH$_2$-$(CH_2)_9$-NH-Boc, HCl, hergestellt analog Beispiel 1 a-b, werden analog Beispiel 1e mit 48 g Z-D-Lys-Phe-OH umgesetzt und aufgearbeitet.
Ausbeute 61,9 g $[\alpha]_D$ : + 1,9° (c = 1, in Methanol)

| $C_{42}H_{64}N_5O_8$ (767,0) | Ber. C 65,77 | H 8,41 | N 9,13 |
| | Gef. C 65,2 | H 8,5 | N 9,0 |

b) Z-D-Lys-Phe-NH-$(CH_2)_9$-NH$_2$ . 2HCl

Durch Abspaltung der Schutzgruppen aus der nach a) hergestellten Verbindung analog Beispiel 1f erhält man die Titelverbindung in chromatographisch einheitlicher Form (A,B) Elementaranalyse korrekt.

Beispiel 41

H-Met(O$_2$)-Glu-His-Phe-D-Lys-Phe-NH-$(CH_2)_9$-NH$_2$ . 3CH$_3$COOH

Man spaltet von dem nach Beispiel 40a erhaltenen Peptidderivat analog Beispiel 8a die Z-Gruppe ab und setzt anschließend mit Z-Phe-OH wie dort beschrieben um. Dann wird analog Beispiel 8b die Z-Gruppe abgespalten und nach den Beispielen 9b und 16a weiterverfahren. Man erhält die geschützte Titelverbindung, von der die Schutzgruppen nach Beispiel 1f abfespalten werden. Aufarbeitung und Reinigung nach Beispiel 11c, Aminosäureanalyse korrekt.

Beispiel 42

H-Met(O$_2$)-Glu-His-Phe-D-Lys-Phe-NH-$(CH_2)_{10}$-NH$_2$ . 3CH$_3$COOH

Man arbeitet nach den Beispielen 40a und 41, setzt jedoch im ersten Schrit NH$_2$-$(CH_2)_{10}$-NH-Boc . HCl ein.
Die Titelverbindung ist nach Reinigung analog Beispiel 11c chromatographisch einheitlich und zeigt eine korrekte Aminosäureanalyse.

Beispiel 43

H-Met(O$_2$)-Glu-His-Phe-D-Lys-Phe-NH-$(CH_2)_6$-NH$_2$ . 3CH$_3$COOH

man arbeitet analog Beispiel 42, setzt jedoch als basischen Rest NH$_2$-$(CH_2)_6$-NH-Boc . HCl ein.

**Patentansprüche**

**Patentansprüche für folgende Vertragsstaaten : BE, CH, DE, FR, GB, IT, LI, LU, NL, SE**

**1.** Verbindung der allgemeinen Formel I

$R^4$-$A^5$-$A^6$-$R^7$ (I)

in welcher bedeuten:
$R^4$ = über N$_\alpha$ gebundenes Benzyloxycarbonyl (Z) oder $R^1$-$A^2$-$A^3$-$A^4$, worin $A^3$, His oder Ala bedeutet,

EP 0 179 332 B1

$A^4$ für Phe steht,

$A^2$ für Glu oder Ala steht und

$R^1$ für Glutaroyl, H-Met(O), H-Met($O_2$), H-Tyr oder Pyroglutamyl steht,

$A^5$ = D-Lys,

$A^6$ = den Rest von Phenylalanin und

$R^7$ = NH-$(CH_2)_n$-$NH_2$, wobei n für eine ganze Zahl von 4-10, steht,

sowie deren physiologisch verträglichen Salze, wobei H-Met(O)-Glu-His-Phe-D-Lys-Phe-NH-$(CH_2)_8$-$NH_2$ ausgenommen ist.

2. Verbindung gemäß Anspruch 1, in welcher n für eine ganze Zahl von 6-10 steht.

3. Verbindung gemäß einem der Ansprüche 1 oder 2, in welcher $R^1$ für H-Met(O)- steht und die Sulfinylgruppe in der R- oder S-Konfiguration vorliegt.

4. Verbindung gemäß einem der Ansprüche 1 oder 2, in welcher $R^1$ für H-Met($O_2$)- steht.

5. Verbindung gemäß einem der Ansprüche 1 oder 2, in welcher $R^1$ für $HO_2$C-$(CH_2)_3$-CO- steht.

6. Verbindung gemäß einem der Ansprüche 1 bis 5, in welcher $R^7$ für -NH-$(CH_2)_8$-$NH_2$ steht.

7. Verbindung gemäß Anspruch 1 mit der Formel H-Met($O_2$)-Glu-His-Phe-D-Lys-Phe-NH-$(CH_2)_8$-$NH_2$ und deren physiologisch verträglichen Salze.

8. Verbindung gemäß Anspruch 1 mit der Formel H-Met($O_2$)-Ala-Ala-Phe-D-Lys-Phe-NH-$(CH_2)_8$-$NH_2$ und deren physiologisch verträglichen Salze.

9. Verbindung gemäß Anspruch 1 mit der Formel HOOC-$(CH_2)_3$-CO-Glu-Ala-Phe-D-Lys-Phe-NH-$(CH_2)_8$-$NH_2$ und deren physiologisch verträglichen Salze.

10. Verbindung gemäß Anspruch 1 mit der Formel H-Met(O)-Glu-His-Phe-D-Lys-Phe-NH-$(CH_2)_6$-$NH_2$ und deren physiologisch verträglichen Salze.

11. Verbindung gemäß Anspruch 1 mit der Formel H-Met($O_2$)-Glu-Ala-Phe-D-Lys-Phe-NH-$(CH_2)_8$-$NH_2$ und deren physiologisch verträglichen Salze.

12. Verbindung gemäß Anspruch 1 mit der Formel H-Tyr-Glu-His-Phe-D-Lys-Phe-NH-$(CH_2)_8$-$NH_2$ und deren physiologisch verträglichen Salze.

13. Verbindung gemäß Anspruch 1 mit der Formel pyro-Glu-Glu-His-Phe-D-Lys-Phe-NH-$(CH_2)_8$-$NH_2$ und deren physiologisch verträglichen Salze.

14. Ein Arzneimittel enthaltend eine wirksame Menge einer Verbindung gemäß einem der Ansprüche 1 bis 13 oder eines ihrer physiologisch verträglichen Salze und einen physiologisch verträglichen Träger.

15. Verbindung gemäß einem der Ansprüche 1 bis 13 zur Anwendung als Heilmittel.

16. Verbindung gemäß einem der Ansprüche 1 bis 13 zur Anwendung als Mittel zur Verbesserung der Lern- und Gedächtnisleistung, bei der Behandlung posttraumatischer und degenerativer Hirnschäden oder zur Verminderung der Hyperemotionalität.

17. Verfahren zur Herstellung einer Verbindung der Formel I gemäß einem der Ansprüche 1 bis 13, dadurch gekennzeichnet, daß man ein Fragment mit N-terminaler freier Aminogruppe kondensiert mit einem Fragment mit C-terminaler freier Carboxylgruppe, wobei in diesen Fragmenten an der Reaktion nicht beteiligte primäre und sekundäre Aminogruppen mit sauer abspaltbaren Schutzgruppen vom Urethantyp und Carboxylgruppen mit sauer abspaltbaren Schutzgruppen vom Estertyp geschützt vorliegen, in den so erhaltenen Verbindungen zum Schutz von Amino- bzw. Carboxylgruppen eingeführte Schutzgruppe sauer abspaltet und die Verbindungen gegebenenfalls in ihre physiologisch verträglichen Salze überführt.

16

**Patentansprüche für folgenden Vertragsstaat : AT**

1. Verfahren zur Herstellung einer Verbindung der allgemeinen Formel I

$R^4$-$A^5$-$A^6$-$R^7$     (I)

in welcher bedeuten:

$R^4$ = über $N_\alpha$ gebundenes Benzyloxycarbonyl (Z) oder $R^1$-$A^2$-$A^3$-$A^4$, worin $A^3$, His oder Ala bedeutet,

$A^4$ für Phe steht,

$A^2$ für Glu oder Ala steht und

$R^1$ für Glutaroyl, H-Met(O), H-Met($O_2$), H-Tyr oder Pyroglutamyl steht,

$A^5$ = D-Lys,

$A^6$ = den Rest von Phenylalanin und

$R^7$ = -NH-$(CH_2)_n$-$NH_2$, wobei n für eine ganze Zahl von 4-10, steht,

sowie deren physiologisch verträglichen Salze, wobei H-Met(O)-Glu-His-Phe-D-Lys-Phe-NH-$(CH_2)_8$-$NH_2$ ausgenommen ist, dadurch gekennzeichnet, daß man ein Fragment mit N-terminaler freier Aminogruppe kondensiert mit einem Fragment mit C-terminaler freier Carboxylgruppe, wobei in diesen Fragmenten an der Reaktion nicht beteiligte primäre und sekundäre Aminogruppen mit sauer abspaltbaren Schutzgruppen vom Urethantyp und Carboxylgruppen mit sauer abspaltbaren Schutzgruppen vom Estertyp geschützt vorliegen, in den so erhaltenen Verbindungen zum Schutz von Amino- bzw. Carboxylgruppen eingeführte Schutzgruppe sauer abspaltet und die Verbindungen gegebenenfalls in ihre physiologisch verträglichen Salze überführt.

2. Verfahren gemäß Anspruch 1, dadurch gekennzeichnet, daß eine Verbindung der Formel I hergestellt wird, in welcher n für eine ganze Zahl von 6-10 steht.

3. Verfahren gemäß einem der Ansprüche 1 oder 2, ddurch gekennzeichnet, daß eine Verbindung der Formel I hergestellt wird, in welcher $R^1$ für H-Met(O)- steht und die Sulfinylgruppe in der R- oder S-Konfiguration vorliegt.

4. Verfahren gemäß einem der Ansprüche 1 oder 2, dadurch gekennzeichnet, daß eine Verbindung der Formel I hergestellt wird, in welcher $R^1$ für H-Met($O_2$)- steht.

5. Verfahren gemäß einem der Ansprüche 1 oder 2, dadurch gekennzeichnet, daß eine Verbindung der Formel I hergestellt wird, in welcher $R^1$ für $HO_2$C-$(CH_2)_3$-CO- steht.

6. Verfahren gemäß einem der Ansprüche 1 bis 5, dadurch gekennzeichnet, daß eine Verbindung der Formel I hergestellt wird, in welcher $R^7$ für -NH-$(CH_2)_8$-$NH_2$ steht.

7. Verfahren gemäß Anspruch 1, dadurch gekennzeichnet, daß H-Met($O_2$)-Glu-His-Phe-D-Lys-Phe-NH-$(CH_2)_8$$NH_2$ und deren physiologisch verträglichen Salze hergestellt werden.

8. Verfahren gemäß Anspruch 1, dadurch gekennzeichnet, daß H-Met($O_2$)-Ala-Ala-Phe-D-Lys-Phe-NH-$(CH_2)_8$-$NH_2$ und deren physiologisch verträglichen Salze hergestellt werden.

9. Verfahren gemäß Anspruch 1, dadurch gekennzeichnet, daß HOOC-$(CH_2)_3$-CO-Glu-Ala-Phe-D-Lys-Phe-NH-$(CH_2)_8$-$NH_2$ und deren physiologisch verträglichen Salze hergestellt werden.

10. Verfahren gemäß Anspruch 1, dadurch gekennzeichnet, daß H-Met(O)-Glu-His-Phe-D-Lys-Phe-NH-$(CH_2)_6$-$NH_2$ und deren physiologisch verträglichen Salze hergestellt werden.

11. Verfahren gemäß Anspruch 1, dadurch gekennzeichnet, daß H-Met($O_2$)-Glu-Ala-Phe-D-Lys-Phe-NH-$(CH_2)_8$-$NH_2$ und dessen physiologisch verträglichen Salze hergestellt werden.

12. Verfahren gemäß Anspruch 1, dadurch gekennzeichnet, daß H-Tyr-Glu-His-Phe-D-Lys-Phe-NH-$(CH_2)_8$-$NH_2$ und dessen physiologisch veträglichen Salze hergestellt werden.

EP 0 179 332 B1

**13.** Verfahren gemäß Anspruch 1, dadurch gekennzeichnet, daß pyro-Glu-Glu-His-Phe-D-Lys-Phe-NH-$(CH_2)_8$-$NH_2$ und dessen physiologisch verträglichen Salze hergestellt werden.

**14.** Verfahren zur Herstellung eines Arzneimittels enthaltend eine wirksame Menge einer Verbindung hergestellt gemäß einem der Ansprüche 1 bis 13 oder eines ihrer physiologisch veträglichen Salze und einen physiologisch veträglichen Träger, dadurch gekennzeichnet, daß man diese in eine geeignete Darreichungsform bringt.

**15.** Verfahren gemäß einem der Ansprüche 1 bis 13 zur Herstellung einer Verbindung der Formel I zur Anwendung als Heilmittel.

**Claims**
**Claims for the following Contracting States : BE, CH, DE, FR, GB, IT, LI, LU, NL, SE**

**1.** A compound of the formula I

$R^4$-$A^5$-$A^6$-$R^7$     (I)

where:

| | |
|---|---|
| $R^4$ | = benzyloxycarbonyl, which is bound via $N_2$ (Z) or $R^1$-$A^2$-$A^3$-$A^4$, in which $A^3$ is His or Ala, |
| $A^4$ | is Phe, |
| $A^2$ | is Glu or Ala and |
| $R^1$ | is glutaroyl, H-Met(O), H-Met($O_2$), H-Tyr or pyroglutamyl, |
| $A^5$ | = D-Lys, |
| $A^6$ | = the radical of phenylalanine and |
| $R^7$ | = -NE-$(CH_2)_n$-$NH_2$, in which n is an integer from 4-10, |

as well as its physiologically tolerated salts, where H-Met(O)-Glu-His-Phe-D-Lys-Phe-NH-$(CH_2)_8$-$NH_2$ is excepted.

**2.** A compound as claimed in claim 1, wherein n is an integer from 6-10.

**3.** A compound as claimed in one of claims 1 or 2, wherein $R^1$ is H-Met(O)- and the sulfinyl group is present in the R or S configuration.

**4.** A compound as claimed in one of claims 1 or 2, wherein $R^1$ is H-Met($O_2$)-.

**5.** A compound as claimed in one of claims 1 or 2, wherein $R^1$ is $HO_2$C-$(CH_2)_3$-CO-.

**6.** A compound as claimed in one of claims 1 to 5, wherein $R^7$ is -NH-$(CH_2)_8$-$NH_2$.

**7.** A compound as claimed in claim 1 with the formula H-Met($O_2$)-Glu-His-Phe-D-Lys-Phe-NH-$(CH_2)_8$-$NH_2$ and its physiologically tolerated salts.

**8.** A compound as claimed in claim 1 with the formula H-Met($O_2$)-Ala-Ala-Phe-D-Lys-Phe-NH-$(CH_2)_8$-$NH_2$ and its physiologically tolerated salts.

**9.** A compound as claimed in claim 1 with the formula HOOC-$(CH_2)_3$-CO-Glu-Ala-Phe-D-Lys-Phe-NH-$(CH_2)_8$-$NH_2$ and its physiologically tolerated salts.

**10.** A compound as claimed in claim 1 with the formula H-Met(O)-Glu-His-Phe-D-Lys-Phe-NH-$(CH_2)_6$-$NH_2$ and its physiologically tolerated salts.

**11.** A compound as claimed in claim 1 with the formula H-Met($O_2$)-Glu-Ala-Phe-D-Lys-Phe-NH-$(CH_2)_8$-$NH_2$ and its physiologically tolerated salts.

**12.** A compound as claimed in claim 1 with the formula H-Tyr-Glu-His-Phe-D-Lys-Phe-NH-$(CH_2)_8$-$NH_2$ and its physiologically tolerated salts.

18

**13.** A compound as claimed in claim 1 with the formula pyro-Glu-Glu-His-Phe-D-Lys-Phe-NH-$(CH_2)_8$-$NH_2$ and its physiologically tolerated salts.

**14.** A pharmaceutical containing an effective quantity of a compound as claimed in one of claims 1 to 13 or one of its physiologically tolerated salts and a physiologically tolerated carrier.

**15.** A compound as claimed in one of claims 1 to 13 to be used as a therapeutic agent.

**16.** A compound as claimed in one of claims 1 to 13 for use as an agent for improving learning and memory performance, in the treatment of post-traumatic and degenerative brain damage, or for decreasing emotional lability.

**17.** A process for preparing a compound of the formula I as claimed in one of claims 1 to 13, wherein a fragment with a free N-terminal amino group is condensed with a fragment with a free C-terminal carboxyl group, where, in these fragments, primary and secondary amino groups that are not participating in the reaction are protected with acid-cleavable protective groups of the urethane type and carboxyl groups are protected with acid-cleavable protective groups of the ester type, and in the compounds thus obtained the protective group used to protect the amino or carboxyl groups is cleaved with acid and the compounds, if required, are converted into their physiologically tolerated salts.

**Claims for the following Contracting State : AT**

**1.** A process for preparing a compound of the formula I

$R^4$-$A^5$-$A^6$-$R^7$     (I)

where:

| | |
|---|---|
| $R^4$ | = benzyloxycarbonyl which is bound via $N_2$ (Z) or $R^1$-$A^2$-$A^3$-$A^4$, in which $A^3$ is His or Ala, |
| $A^4$ | is Phe, |
| $A^2$ | is Glu or Ala and |
| $R^1$ | is glutaroyl, H-Met(O), H-Met($O_2$), H-Tyr or pyroglutamyl, |
| $A^5$ | = D-Lys, |
| $A^6$ | = the radical of phenylalanine and |
| $R^7$ | = -NH-$(CH_2)_n$-$NH_2$, in which n is an integer from 4-10, |

as well as its physiologically tolerated salts, where H-Met(O)-Glu-His-Phe-D-Lys-Phe-NH-$(CH_2)_8$-$NH_2$ is excepted, wherein a fragment with a free N-terminal amino group is condensed with a fragment with a free C-terminal carboxyl group, where, in these fragments, primary and secondary amino groups that are not participating in the reaction are protected with acid-cleavable protective groups of the urethane type and carboxyl groups are protected with acid-cleavable protective groups of the ester type, and in the compounds thus obtained the protective group used to protect the amino or carboxyl groups is cleaved with acid and the compounds, if required, converted into their physiologically tolerated salts.

**2.** The process as claimed in claim 1, wherein a compound of the formula I is prepared, in which n is an integer from 6-10.

**3.** Process as claimed in one of claims 1 or 2, wherein a compound of the formula I is prepared, in which $R^1$ is H-Met(O)- and the sulfinyl group is present in the R or S configuration.

**4.** The process as claimed in one of claims 1 or 2, wherein a compound of the formula I is prepared, in which $R^1$ is H-Met($O_2$)-.

**5.** The process as claimed in one of claims 1 or 2, wherein a compound of the formula I is prepared, in which $R^1$ is $HO_2$C-$(CH_2)_3$-CO-.

**6.** The process as claimed in one of claims 1 to 5, wherein a compound of the formula I is prepared, in which $R^7$ is -NH-$(CH_2)_8$-$NH_2$.

**7.** The process as claimed in claim 1, wherein H-Met($O_2$)-Glu-His-Phe-D-Lys-Phe-NH-$(CH_2)_8$-$NH_2$ and its

EP 0 179 332 B1

physiologically tolerated salts are prepared.

8. The process as claimed in claim 1, wherein H-Met($O_2$)-Ala-Ala-Phe-D-Lys-Phe-NH-$(CH_2)_8$-$NH_2$ and its physiologically tolerated salts are prepared.

9. The process as claimed in claim 1, wherein HOOC-$(CH_2)_3$-CO-Glu-Ala-Phe-D-Lys-Phe-NH-$(CH_2)_8$-$NH_2$ and its physiologically tolerated salts are prepared.

10. The process as claimed in claim 1, wherein H-Met(O)-Glu-His-Phe-D-Lys-Phe-NH-$(CH_2)_8$-$NH_2$ and its physiologically tolerated salts are prepared.

11. The process as claimed in claim 1, wherein H-Met($O_2$)-Glu-Ala-Phe-D-Lys-Phe-NH-$(CH_2)_8$-$NH_2$ and its physiologically tolerated salts are prepared.

12. The process as claimed in claim 1, wherein H-Tyr-Glu-His-Phe-D-Lys-Phe-NH-$(CH_2)_8$-$NH_2$ and its physiologically tolerated salts are prepared.

13. The process as claimed in claim 1, wherein pyro-Glu-Glu-His-Phe-D-Lys-Phe-NH-$(CH_2)_8$-$NH_2$ and its physiologically tolerated salts are prepared.

14. A process for the preparation of a pharmaceutical containing an effective quantity of a compound prepared as claimed in one of claims 1 to 13 or one of its physiologically tolerated salts and a physiologically tolerated carrier, wherein it is brought into a suitable form for administration.

15. The process as claimed in one of claims 1 to 13 for the preparation of a compound of the formula I to be used as a therapeutic agent.

**Revendications**
**Revendications pour les Etats contractants suivants : BE, CH, DE, FR, GB, IT, LI, LU, NL, SE**

1. Composé de formule générale I

$R^4$-$A^5$-$A^6$-$R^7$     (I),

dans laquelle
    $R^4$    représente benzyloxycarbonyle (Z) ou $R^1$-$A^2$-$A^3$-$A^4$, où
    $A^3$    est His ou Ala,
    $A^4$    est Phe,
    $A^2$    est Glu ou Ala,
    $R^1$    est un groupe glutaryle, H-Met(O), H-Met($O_2$), HTyr ou pyroglutamyle,
    $A^5$    est D-Lys,
    $A^6$    est le reste de la phénylalanine, et
    $R^7$    représente -NH-$(CH_2)_n$-$NH_2$, n étant un nombre entier de 4 à 10,
ainsi que ses sels physiologiquement compatibles, à l'exclusion de H-Met(O)-Glu-His-Phe-D-Lys-Phe-NH-$(CH_2)_8$-$NH_2$.

2. Composé selon la revendication 1, dans lequel n est un nombre entier valant de 6 à 10.

3. Composé selon une des revendications 1 ou 2, dans lequel $R^1$ représente H-Met(O)- et le groupe sulfinyle présente aussi bien la configuration R que S.

4. Composé selon une des revendications 1 ou 2, dans lequel $R^1$ représente H-Met($O_2$)-.

5. Composé selon une des revendications 1 ou 2, dans lequel $R^1$ représente $HO_2$C-$(CH_2)_3$-CO-.

6. Composé selon une des revendications 1 à 5, dans lequel $R^7$ représente -NH-$(CH_2)_8$-$NH_2$.

7. Composé selon la revendication 1, de formule H-Met($O_2$)-Glu-His-Phe-D-Lys-Phe-NH-$(CH_2)_8$-$NH_2$, et

20

ses sels physiologiquement compatibles.

**8.** Composé selon la revendication 1, de formule H-Met(O₂)-Ala-Ala-Phe-D-Lys-Phe-NH-(CH₂)₈-NH₂, et ses sels physiologiquement compatibles.

**9.** Composé selon la revendication 1, de formule HOOC-(CH₂)₃-CO-Glu-Ala-Phe-D-Lys-Phe-NH-(CH₂)₈-NH₂, et ses sels physiologiquement compatibles.

**10.** Composé selon la revendication 1, de formule H-Met(O)-Glu-His-Phe-D-Lys-Phe-NH-(CH₂)₆-NH₂, et ses sels physiologiquement compatibles.

**11.** Composé selon la revendication 1, de formule H-Met(O₂)-Glu-Ala-Phe-D-Lys-Phe-NH-(CH₂)₈-NH₂, et ses sels physiologiquement compatibles.

**12.** Composé selon la revendication 1, de formule H-Tyr-Glu-His-Phe-D-Lys-Phe-NH-(CH₂)₈-NH₂, et ses sels physiologiquement compatibles.

**13.** Composé selon la revendication 1, de formule pyro-Glu-Glu-His-Phe-D-Lys-Phe-NH-(CH₂)₈-NH₂, et ses sels physiologiquement compatibles.

**14.** Médicament contenant une quantité active d'un composé selon l'une des revendications 1 à 13 ou d'un de ses sels physiologiquement compatibles, et un véhicule physiologiquement compatible.

**15.** Composé selon une des revendications 1 à 13, utilisé comme médicament.

**16.** Composé selon une des revendications 1 à 13, utilisé comme médicament pour améliorer les capacités d'étude et de mémoire, pour traiter les atteintes cérébrales post-traumatiques et dégénératives ou pour diminuer l'hyperémotivité.

**17.** Procédé de préparation d'un composé de formule I selon l'une des revendications 1 à 13, caractérisé en ce qu'on condense un fragment à groupe amino libre N-terminal avec un fragment à groupe carboxyle libre C-terminal, les groupes amino primaires et secondaires de ces fragments qui ne prennent pas part à la réaction étant protégés par des groupes protecteurs clivables en milieu acide, du type uréthane, et les groupes carboxyle étant protégés par des groupes protecteurs clivables en milieu acide, du type ester, on clive en milieu acide les groupes protecteurs introduits dans les composés ainsi obtenus pour la protection des groupes amino ou carboxyle et on transforme éventuellement ces composés en leurs sels physiologiquement compatibles.

**Revendications pour l'Etat contractant suivant : AT**

**1.** Procédé de préparation d'un composé de formule générale I

$R^4$-$A^5$-$A^6$-$R^7$     (I),

dans laquelle
$R^4$     représente benzyloxycarbonyle (Z) ou $R^1$-$A^2$-$A^3$-$A^4$, où
$A^3$     est His ou Ala,
$A^4$     est Phe,
$A^2$     est Glu ou Ala,
$R^1$     est un groupe glutaryle, H-Met(O), H-Met(O₂), HTyr ou pyroglutamyle,
$A^5$     est D-Lys,
$A^6$     est le reste de la phénylalanine, et
$R^7$     représente -NH-(CH₂)ₙ-NH₂, n étant un nombre entier de 4 à 10,
ainsi que ses sels physiologiquement compatibles, à l'exclusion de H-Met(O)-Glu-His-Phe-D-Lys-Phe-NH-(CH₂)₈-NH₂, caractérisé en ce qu'on condense un fragment à groupe amino libre N-terminal avec un fragment à groupe carboxyle libre C-terminal, les groupes amino primaires et secondaires de ces fragments qui ne prennent pas part à la réaction étant protégés par des groupes protecteurs clivables en milieu acide, du type uréthane, et les groupes carboxyle étant protégés par des groupes protecteurs

EP 0 179 332 B1

clivables en milieu acide, du type ester, on clive en milieu acide les groupes protecteurs introduits dans les composés ainsi obtenus pour la protection des groupes amino ou carboxyle et on transforme éventuellement ces composés en leurs sels physiologiquement compatibles.

2. Procédé selon la revendication 1, caractérisé en ce qu'on prépare un composé de formule I dans laquelle n est un nombre entier valant de 6 à 10.

3. Procédé selon une des revendications 1 ou 2, caractérisé en ce qu'on prépare un composé de formule I dans laquelle $R^1$ représente H-Met(O)- et le groupe sulfinyle présente aussi bien la configuration R que S.

4. Procédé selon une des revendications 1 ou 2, caractérisé en ce qu'on prépare un composé de formule I dans laquelle $R^1$ représente H-Met($O_2$)-.

5. Procédé selon une des revendications 1 ou 2, caractérisé en ce qu'on prépare un composé de formule I dans laquelle $R^1$ représente $HO_2C$-$(CH_2)_3$-CO-.

6. Procédé selon une des revendications 1 à 5, caractérisé en ce qu'on prépare un composé de formule I dans laquelle $R^7$ représente -NH-$(CH_2)_8$-$NH_2$.

7. Procédé selon la revendication 1, caractérisé en ce qu'on prépare H-Met($O_2$)-Glu-His-Phe-D-Lys-Phe-NH-$(CH_2)_8$-$NH_2$ et ses sels physiologiquement compatibles.

8. Procédé selon la revendication 1, caractérisé en ce qu'on prépare H-Met($O_2$)-Ala-Ala-Phe-D-Lys-Phe-NH-$(CH_2)_8$-$NH_2$ et ses sels physiologiquement compatibles.

9. Procédé selon la revendication 1, caractérisé en ce qu'on prépare HOOC-$(CH_2)_3$-CO-Glu-Ala-Phe-D-Lys-Phe-NH-$(CH_2)_8$-$NH_2$ et ses sels physiologiquement compatibles.

10. Procédé selon la revendication 1, caractérisé en ce qu'on prépare H-Met(O)-Glu-His-Phe-D-Lys-Phe-NH-$(CH_2)_6$-$NH_2$ et ses sels physiologiquement compatibles.

11. Procédé selon la revendication 1, caractérisé en ce qu'on prépare H-Met($O_2$)-Glu-Ala-Phe-D-Lys-Phe-NH-$(CH_2)_8$-$NH_2$ et ses sels physiologiquement compatibles.

12. Procédé selon la revendication 1, caractérisé en ce qu'on prépare H-Tyr-Glu-His-Phe-D-Lys-Phe-NH-$(CH_2)_8$-$NH_2$ et ses sels physiologiquement compatibles.

13. Procédé selon la revendication 1, caractérisé en ce qu'on prépare pyro-Glu-Glu-His-Phe-D-Lys-Phe-NH-$(CH_2)_8$-$NH_2$ et ses sels physiologiquement compatibles.

14. Procédé de fabrication d'un médicament contenant une quantité active d'un composé selon l'une des revendications 1 à 13 ou d'un de ses sels physiologiquement compatibles, et un véhicule physiologiquement compatible, caractérisé en ce qu'on le met sous une forme d'administration appropriée.

15. Procédé selon l'une des revendications 1 à 13, pour la préparation d'un composé de formule I utilisé comme médicament.